# EUROPEAN PATENT APPLICATION

(11) **EP 4 721 684 A1**
(43) Date of publication of application: **08.04.2026**
(21) Application number: 24815328.0
(22) Date of filing: 22.05.2024
(51) Int. Cl.: A61B 18/12

(54) **HIGH-FREQUENCY TREATMENT APPARATUS AND HIGH-FREQUENCY TREATMENT METHOD**

(30) Priority: 27.05.2023 JP 2023087332
(71) Applicant: Kabushiki Kaisha Top, Adachi-ku, Tokyo 120-0035 (JP)
(72) Inventor: IKEUCHI Atsushi, Tokyo 120-0035 (JP)
(74) Representative: Pritzlaff, Stefanie Lydia
(86) International application number: PCT/JP2024/018812
(87) International publication number: WO 2024/247844

(57) **Abstract**

To provide a high-frequency treatment apparatus and a high-frequency treatment method capable of more efficiently performing high-frequency treatment. A high-frequency treatment apparatus (1) is provided with a high-frequency output unit (40) which outputs high-frequency power, electrodes (20, 30) which are connected to the high-frequency output unit (40) and arranged on a treatment object, an impedance measurement unit (50) which is connected to the electrodes (20, 30) and measures impedance by outputting an impedance measurement signal, and a control unit (100) which controls the impedance measurement unit (50) so as to continue intermittently outputting the impedance measurement signal before and after the start of treatment with high-frequency power.

## Description

The present invention relates to an apparatus and method for high-frequency treatment which perform various treatments by passing a high-frequency current to an object to be treated.

Conventionally, in the field of medicine, high-frequency treatment in which an electrode is arranged in the body of a human being, an animal, or the like, and a high-frequency current is passed through the electrode to ablate tissue (ablation) or the like has been widely used. In recent years, a technique for performing nerve block by high-frequency treatment has attracted attention as one of pain treatments. Nerve block by high-frequency treatment has the advantages of having fewer side effects on surrounding tissue and lasting effects for a long period of time compared with a technique using a conventional medicine.

There are two types of nerve block by high-frequency treatment: the high-frequency thermocoagulation method and the pulsed high-frequency method. In the high-frequency thermocoagulation method, a part of nerve tissue is thermocoagulated by a high-frequency current flowing from an electrode at a temperature of about 80°C for several minutes to block pain signals. In the pulsed high-frequency method, a high-frequency current is applied intermittently (in the form of a pulse signal) while the temperature around the electrode is kept below 42-45°C. The electric field generated around the electrode affects the nerve and blocks pain signals without degenerating the nerve tissue.

When performing such high-frequency treatment, the impedance between the electrodes through which the high-frequency current flows is usually measured before and during the treatment. By measuring this impedance, it is possible to detect defects such as electrode disconnection or mounting failure of a counter electrode plate. In addition, since the impedance varies depending on the part in a living body, it is possible to estimate the position of the electrode tip (the part where the high-frequency current is output) based on the change of the impedance measured when the electrode is inserted into the treatment object (For example, see non-patent document 1 or 2.).

### [Citation List]

### [Non Patent Literature 1]

Koichi Fujii and Shinsuke Hamaguchi, "Therapeutic device: high-frequency thermocoagulation device ", Medical Instrumentation, Japanese Society of Medical Devices, 2020, Vol. 90, No. 3, p. 285-289

### [Non Patent Literature 2]

Mikio Fukui, "Pulsed high-frequency (PRF) up to date ", Japanese Pain Clinic Society Journal, Japanese Pain Clinic Society, 2013, Vol. 20, No. 1, p. 1-7

### [Problem to be solved]

However, the measurement of impedance in a conventional apparatus is limited to merely measuring impedance, and it is not possible to perform a high-frequency treatment more efficiently by utilizing the measurement of impedance.

It is an object of the present invention to provide a high-frequency treatment apparatus and high-frequency treatment method capable of performing a high-frequency treatment more efficiently.

### [Solution to the problem]

The high-frequency treatment apparatus of the present invention is characterized by comprising: a high-frequency output unit for outputting high-frequency power; an electrode connected to the high-frequency output unit and arranged on a treatment object; an impedance measurement unit connected to the electrode and outputting an impedance measurement signal to measure impedance; and a control unit for controlling the impedance measurement unit so as to continue outputting the impedance measurement signal intermittently before and after the start of the treatment with the high-frequency power.

In the high-frequency treatment method for performing treatment by outputting high-frequency power to an electrode arranged in a treatment object, the intermittent output of an impedance measurement signal for measuring impedance is continued from a state before starting the treatment to a state after starting the treatment.

According to the high-frequency treatment apparatus and the high-frequency treatment method of the present invention, the impedance measurement signal can be utilized for other purposes by appropriately setting the mode of output of the impedance measurement signal before starting the treatment. In addition, since the impedance is conventionally calculated from the voltage measurement value and the current measurement value of the high-frequency power after starting the treatment, there has been a problem that an inaccurate impedance is easily calculated due to the difficulty of direct measurement of the high-frequency current with high accuracy. However, according to the present invention, since the impedance can be measured by the impedance measurement signal even after starting the treatment, the impedance can be measured with high accuracy. That is, the high-frequency treatment can be performed more efficiently.

In the high-frequency treatment apparatus of the present invention, it is preferable that the control unit controls the impedance measurement unit so as to output the impedance measurement signal in a mode in which the motor nerve is stimulated before starting the treatment.

This makes it possible to perform nerve exploration by the impedance measurement signal, so that the high-frequency treatment can be performed more efficiently. That is, nerve exploration using a dedicated stimulation signal is usually performed before the execution of the high-frequency treatment in order to confirm that there is no motor nerve affected by the high-frequency treatment, but this can be omitted. In addition, even when nerve exploration using the dedicated stimulation signal is performed, the nerve exploration is performed again during standby before the start of the treatment, so that the safety of the treatment can be further enhanced.

In the high-frequency treatment apparatus of the present invention, it is preferable that the control unit controls the impedance measurement unit so that after the start of the treatment, the impedance measurement signal is output in a manner different from the state before the start of the treatment.

According to this, for example, before the start of the treatment, the impedance measurement signal is output in a manner in which the motor nerve is stimulated, and after the start of the treatment, the impedance measurement signal is output in synchronization with the intermittent output of the high-frequency power, so that the high-frequency treatment can be performed more efficiently.

In the high-frequency treatment apparatus of the present invention, it is preferable that the high-frequency output unit intermittently outputs high-frequency power after the start of the treatment, and that the control unit controls the impedance measurement unit so as to output the impedance measurement signal during a stop period of the output of the high-frequency power after the start of the treatment.

According to this arrangement, the connection to the electrode is switched between the high-frequency output unit and the impedance measurement unit, so that the impedance can be efficiently measured by the impedance measurement signal even after the start of the treatment.

In the high-frequency treatment apparatus of the present invention, it is preferable that the control unit has an alarm device which is controlled by the control unit to issue an alarm, and that the control unit controls the alarm device so as to issue an alarm when the impedance measured by the impedance measurement unit is a value indicating a short-circuit state.

According to this arrangement, it is possible to check the continuity of the electrode by utilizing the impedance measurement signal, so that the high-frequency treatment can be performed more efficiently.

### [Effect of Invention]

According to the high-frequency treatment apparatus and the high-frequency treatment method of the present invention, it is possible to obtain an excellent effect that the high-frequency treatment can be performed more efficiently.

### [Detailed Description of the Drawings]

[FIG. 1] FIG. 1 is a schematic diagram showing an external appearance of a radiofrequency treatment apparatus according to an embodiment of the present invention;
[FIG. 2] FIG. 2 is a block diagram showing a schematic of an internal configuration of a radiofrequency treatment apparatus;
[FIG. 3] FIG. 3 is a time chart outlining an operation of a high-frequency treatment apparatus in a menu mode; [FIG. 4] FIGS. 4 A-D are schematic diagrams showing connection states in a menu mode;
[FIG. 5] FIGS. 5 A-D are schematic diagrams showing connection states in a monopolar configuration;
[FIG. 6] FIGS. 6 A-D are schematic diagrams showing connection states in a monopolar configuration;
[FIG. 7] FIGS. 7 A-C are schematic diagrams showing connection states in a bipolar configuration;
[FIG. 8] FIGS. 8 A-C are schematic diagrams showing connection states in a tripolar configuration;
[FIG. 9] FIGS. 9 A and B are schematic diagrams showing connection states in a quad-polar configuration;
[FIG. 10] FIG. 10 is a time chart outlining the operation of a high-frequency treatment apparatus in stimulation mode;
[FIG. 11] FIG. 11 is a time chart outlining the operation of a high-frequency treatment apparatus in region mode; [FIG. 12] FIGS. 12 A and B are schematic diagrams showing connection states during impedance measurement during bipolar processing;
[FIG. 13] FIGS. 13 A-C are schematic diagrams showing connection states during impedance measurement during tripolar processing;
[FIG. 14] FIGS. 14 A-C are schematic diagrams showing connection states during impedance measurement during quad-polar processing;
[FIG. 15] FIG. 15 is a time chart outlining operation of a high-frequency treatment apparatus in PRF mode;

### [Embodiments of the invention]

Hereinafter, an embodiment of the present invention will be described with reference to the accompanying drawings.

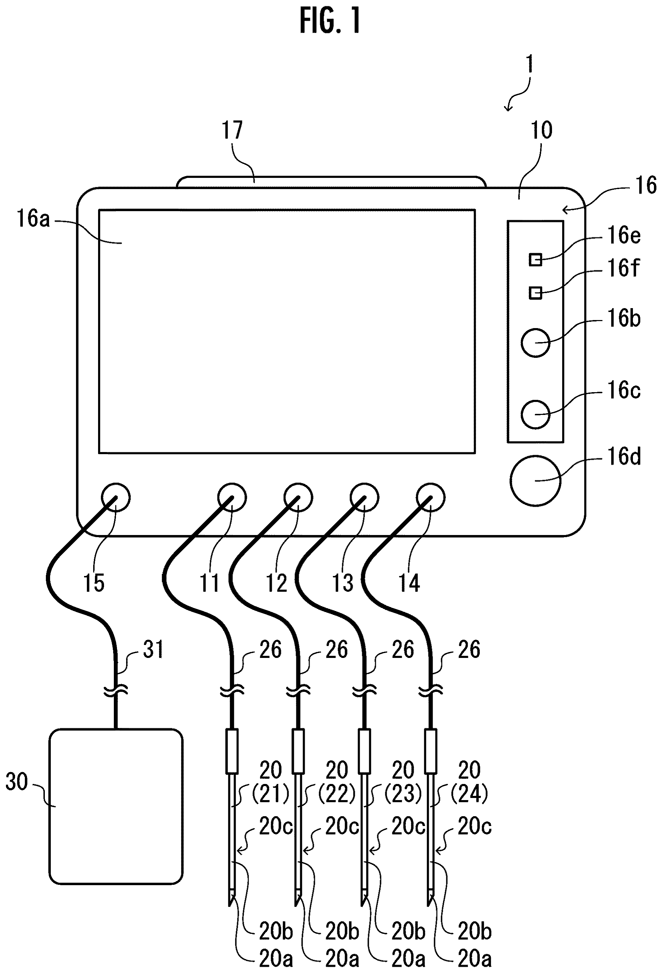

FIG. 1 is a schematic view showing an appearance of a high-frequency treatment apparatus 1 according to an embodiment of the present invention. The high-frequency treatment apparatus 1 of the present embodiment applies a high-frequency voltage to a living body such as a human being or an animal to be treated, and a high-frequency current flows in the living body to perform treatment by either a high-frequency thermocoagulation method or a pulsed high-frequency method. As shown in FIG. 1, the high-frequency treatment apparatus 1 includes a body 10, 4 electrodes 20 (21, 22, 23, and 24) connected to the body 10, and a counter electrode plate 30 connected to the body 10.

The body 10 accommodates or supports internal structures described later. On the front surface of the main body 10, 4 electrode connectors 11-14 to which electrodes 20 are connected and a counter electrode plate connector 15 to which a counter electrode plate 30 is connected are provided at a lower portion. On the front surface of the main body 10, an operation unit 16 for receiving an operation by a user is also provided. The operation unit 16 is composed of a touch panel display 16a for receiving an input operation such as various settings and displaying various information, a start button 16b for receiving a start operation of a treatment, a stop button 16c for receiving an end operation of the treatment, a control knob 16d for performing an output start operation and an output voltage adjustment operation at the time of manual operation, an output indicator 16e which is turned on during the output of high-frequency power, and an alarm indicator 16f which is turned on when an alarm is generated.

On the upper portion of the main body 10, a handle 17 for carrying the main body 10 is provided. Although not shown, a power connector connected to a commercial AC power source for receiving power, a power switch for receiving a power-on operation, and a USB connector for connecting an external storage means are provided on the rear surface of the main body 10. A speaker (alarm) for outputting a buzzer sound is provided inside the main body 10 together with a drive circuit.

The electrode 20 is inserted into an object to be treated in a high-frequency treatment to allow a high-frequency current to flow into the object. In this embodiment, the electrode 20 is formed into a needle tube that can be directly punctured into a human body or the like. Most of the electrode 20 is an insulating portion 20b coated with insulation except for the non-insulating portion 20a at the tip, and the high-frequency current is output from the non-insulating portion 20a. The electrode 20 also incorporates a thermocouple 20c for measuring the temperature around the electrode 20 during the treatment.

The electrode 20 is electrically connected to the main body 10 via an electrode cable 26 and electrode connectors 11-14. In this embodiment, a maximum of 4 electrodes 20 (21-24) can be used simultaneously. The electrode 20 may have other shapes, such as a rod-shaped electrode inserted into a needle tube or catheter, or a pad-shaped electrode arranged on the surface of a living body. The thermocouple 20c may be provided separately from the electrode 20.

The counter electrode plate 30 is a plate-shaped electrode which is attached to the surface of the object to be treated and is used for passing a high-frequency current to and from the electrode 20. In this embodiment, the high-frequency current can be passed not only between the electrode 20 and the counter electrode plate 30 but also between the electrodes 20 (for example, between the electrodes 21 and 22). The counter electrode plate 30 is electrically connected to the main body 10 via the counter electrode plate cable 31 and the counter electrode plate connector 15. In this embodiment, the counter electrode plate 30 is formed in a rectangular flat plate shape, but the counter electrode plate 30 may have other shapes.

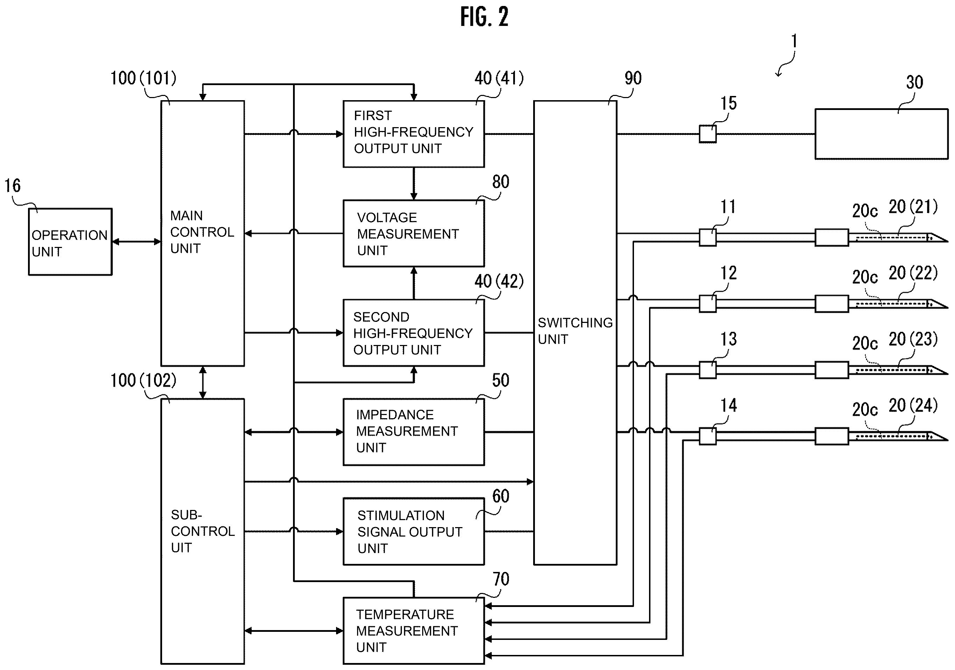

FIG. 2 is a block diagram schematically showing an internal configuration of the high-frequency treatment apparatus 1. As shown in FIG. 2, the high-frequency treatment apparatus 1 includes a high-frequency output unit 40 (a first high-frequency output unit 41 and a second high-frequency output unit 42), an impedance measurement unit 50, a stimulation signal output unit 60, a temperature measurement unit 70, a voltage measurement unit 80, and a control unit 100 (a main control unit 101 and a sub control unit 102).

The high-frequency output unit 40 generates and outputs high-frequency power of a preset frequency (For example, 470-490 kHz) and a voltage (For example, 18-80Vrms) based on a control signal from the main control unit 101 based on power supplied from the commercial AC power supply. In this embodiment, by providing two high-frequency output units 40, that is, the first high-frequency output unit 41 and the second high-frequency output unit 42, it is possible to flow a high-frequency current while stably performing output control at the two electrode sets (For example, an electrode set of electrodes 21 and 22 and an electrode set of electrodes 23 and 24) at the same time.

The high-frequency output unit 40 is connected to the electrode connector 11-14 and the counter electrode plate connector 15 via the switching unit 90. Therefore, the electrode 20 is connected to the high-frequency output unit 40 via the electrode connector 11-14 and the switching unit 90, and the counter electrode plate 30 is connected to the high-frequency output unit 40 via the counter electrode plate connector 15 and the switching unit 90. Each of the high-frequency output units 40 is composed of a known circuit having a transformer, whereby the treatment target is insulated from a commercial AC power supply.

The impedance measurement unit 50 is composed of a known circuit or the like, generates and outputs an impedance measurement signal composed of a relatively weak AC power, and measures the impedance between the electrode 20 and the counter electrode plate 30 or between the electrodes 20, that is, the impedance of a portion where a high-frequency current flows in the treatment target. The impedance measurement unit 50 is connected to the electrode connectors 11-14 and the counter electrode plate connector 15 via the switching unit 90. Accordingly, the electrode 20 is connected to the impedance measurement unit 50 via the electrode connectors 11-14 and the switching unit 90, and the counter electrode plate 30 is connected to the impedance measurement unit 50 via the counter electrode plate connector 15 and the switching unit 90.

The impedance measurement unit 50 outputs an impedance measurement signal under the control of the sub-control unit 102, samples the impedance measurement signal flowing in the treatment target at a predetermined sampling period, discretizes the signal, and performs a discrete Fourier transform to calculate the values of the real part and the imaginary part of the complex impedance. These calculated values are transmitted to the sub-control unit 102. In this embodiment, the impedance measurement signal is a sine wave with a frequency of 50 kHz, the maximum output current is set to about 4 mA, and the maximum output voltage is set to about 500 mVrms. Although the details will be described later, in this embodiment, the intermittent output of the impedance measurement signal is continued before and after the start of the treatment, so that the high-frequency treatment can be efficiently performed.

The stimulation signal output unit 60 is composed of a known circuit or the like, and generates and outputs a stimulation signal for performing nerve exploration. Normally, nerve exploration is performed before performing the high-frequency treatment, and the presence or absence of nerves (sensory nerves and motor nerves) around the electrode 20 is explored. The stimulation signal output unit 60 is connected to the electrode connectors 11-14 and the counter electrode plate connector 15 via the switching unit 90. Therefore, the electrode 20 is connected to the stimulation signal output unit 60 via the electrode connectors 11-14 and the switching unit 90, and the counter electrode plate 30 is connected to the stimulation signal output unit 60 via the counter electrode plate connector 15 and the switching unit 90.

The stimulation signal output unit 60 operates under the control of the sub-control unit 102, and sends a stimulation signal into the treatment object via the electrode 20. The user confirms the presence of the sensory nerve around the electrode 20 when the treatment object perceives the stimulation signal, and confirms the presence of the motor nerve around the electrode 20 when the muscle of the treatment object contracts in response to the stimulation signal. In this embodiment, the stimulation signal is a bipolar rectangular wave having a predetermined pulse width, and is intermittently output at a predetermined frequency.

The pulse width of the stimulation signal can be selected from 0.1msec, 0.2msec, 0.5msec, and 1.0msec, and the frequency can be selected from 10 Hz, 20 Hz, 50 Hz, 75 Hz, 100 Hz, 150 Hz, 180 Hz, and 200 Hz for sensory nerve exploration, and from 1 Hz, 2 Hz, and 5 Hz for motor nerve exploration.

The temperature measuring unit 70 is composed of a known circuit or the like, and measures the temperature around the electrode 20 by the thermocouple 20c. In this embodiment, the temperature measuring unit 70 is connected to the thermocouple 20c in the electrode 20 via the electrode connectors 11-14, but a connector for the thermocouple 20c may be provided separately.

The temperature measuring unit 70 operates under the control of the sub-control unit 102, generates a temperature measuring signal (E.G. 25 mV/°C) based on a signal received from the thermocouple 20c, and transmits it to the sub-control unit 102. The temperature measuring unit 70 also outputs an output stop signal to the high-frequency output unit 40 and the main control unit 101 when a temperature abnormality occurs (when the temperature measured value is higher than the set temperature by 7°C. or more). The high-frequency output unit 40 which has received the output stop signal stops outputting the high-frequency power. The main control unit 101 which has received the output stop signal turns on the alarm indicator 16f and outputs an alarm buzzer sound from a speaker.

The voltage measuring unit 80 is composed of a known circuit or the like and measures the voltage of the high-frequency power output from the high-frequency output unit 40. The voltage measuring unit 80 individually measures the voltage of the high-frequency power output from the first high-frequency output unit 41 and the second high-frequency output unit 42, generates a voltage measurement signal based on the voltage measurement signal, and transmits the voltage measurement signal to the main control unit 101.

The switching unit 90 is composed of a plurality of lead relays or semiconductor switches or the like and switches the connection states of the first high-frequency output unit 41, the second high-frequency output unit 42, the impedance measurement unit 50, and the stimulation signal output unit 60 with the electrode connectors 11-14 and the counter electrode plate connector 15.

Specifically, the switching unit 90 operates under the control of the sub-control unit 102 and switches which of the first high-frequency output unit 41, the second high-frequency output unit 42, the impedance measurement unit 50, and the stimulation signal output unit 60 is connected to the electrode connectors 11-14 and the counter electrode plate connector 15. That is, the switching unit 90 switches which of the high-frequency power, the impedance measurement signal, and the stimulation signal is output to the electrodes 21-24 and the counter electrode 30 connected to the electrode connectors 11-14 and the counter electrode connector 15. The switching unit 90 also operates under the control of the sub-control unit 102 and switches which of the combinations of the electrodes 21-24 and the counter electrode 30 connected to the electrode connectors 11-14 and the counter electrode connector 15 the high-frequency current, the impedance measurement signal, and the stimulation signal flows.

The control unit 100 executes the high-frequency treatment by controlling each part of the high-frequency treatment apparatus 1. In this embodiment, the control unit 100 comprises a main control unit 101 for controlling the high-frequency output unit 40 and the operation unit 16, and a sub-control unit 102 for controlling the impedance measurement unit 50, the stimulation signal output unit 60, the temperature measurement unit 70, and the switching unit 90. As described above, the sub-control unit 102 for controlling a part provided closer to the treatment target than the high-frequency output unit 40 is provided separately from the main control unit 101, so that the treatment target can be more reliably insulated from the commercial AC power supply.

The main control unit 101 has a known configuration such as a CPU, a ROM, a RAM, and an auxiliary storage device. The main control unit 101 starts and ends the high-frequency treatment based on an input operation received by the operation unit 16 and information stored in the ROM or the like. The main control unit 101 controls the output of the high-frequency output unit 40 during the treatment. Specifically, the main control unit 101 controls the output so that the temperature measurement value received from the sub-control unit 102 is substantially equal to the set temperature by using the output voltage as the operation quantity in the high-frequency thermal solidification method and the output pulse width as the operation quantity in the pulsed high-frequency method (The output voltage is fixed to the voltage set value.). In both the high-frequency thermal solidification method and the pulsed high-frequency method, the main control unit 101 controls the ON/OFF of the output of the high-frequency power at a set cycle, although the details will be described later.

During the processing, the main control unit 101 causes the touch panel display 16a to display information such as elapsed time, temperature measurement value, impedance measurement value, voltage measurement value, and current measurement value. In this embodiment, the current measurement value is calculated from the voltage measurement value and the impedance measurement value by the main control unit 101.

The sub-control unit 102 has a known configuration such as a CPU, ROM, and RAM. The sub-control unit 102 calculates a temperature measurement value based on the temperature measurement signal received from the temperature measurement unit 70, and transmits the calculated temperature measurement value to the main control unit 101. The sub-control unit 102 also calculates an impedance measurement value based on the values of the real part and imaginary part of the complex impedance received from the impedance measurement unit 50 and a calibration value generated in advance, and transmits the calculated value to the main control unit 101.

The calibration value is generated by the sub-control unit 102 every time the high-frequency treatment apparatus 1 is started. When the high-frequency treatment apparatus 1 is powered on, the sub-control unit 102 causes the impedance measurement unit 50 to measure impedances of a built-in fixed resistance of 100 Ω and a fixed resistance of 1 kΩ. The low-resistance calibration value is generated based on the measurement result of the fixed resistance of 100 Ω, and the high-resistance calibration value is generated based on the measurement result of the fixed resistance of 1 kΩ, and stored in a RAM. The impedance measurement value is calculated by using the high-resistance calibration value in the first calculation, and in subsequent calculations, a predetermined value (300 Ω or 400 Ω) is set as a threshold value, and either the low-resistance calibration value or the high-resistance calibration value is used based on the previous calculation result.

The sub-control unit 102 controls on/off of a plurality of switches provided in the switching unit 90, and switches the connection states of the first high-frequency output unit 41, the second high-frequency output unit 42, the impedance measurement unit 50, the stimulation signal output unit 60, and the electrode connectors 11-14 and the counter electrode plate connector 15. Specifically, the sub-control unit 102 causes the switching unit 90 to switch the connection state based on the connection state of the electrode 20 detected from the electrode connectors 11-14 and the mode information and output format information received from the main control unit 101.

Next, the operation of the high-frequency treatment apparatus 1 will be described.

When the high-frequency treatment apparatus 1 is powered on, the main control unit 101 and the sub-control unit 102 firstly perform various self-checks and initial settings. At this time, the sub-control unit 102 generates a low-resistance calibration value and a high-resistance calibration value for impedance measurement value calculation. When the self-checks and initial settings are completed, the main control unit 101 shifts to the menu mode.

In the menu mode, the user name and the ID of the treatment target are set based on an input operation to the touch panel display 16a. When the electrode 20 and the counter electrode 30 are connected to the electrode connector 11-14 and the counter electrode connector 15, the sub-control unit 102 starts temperature measurement by the temperature measurement unit 70 and impedance measurement by the impedance measurement unit 50. The main control unit 101 displays the temperature measurement value and the impedance measurement value for each electrode 20 on the touch panel display 16a.

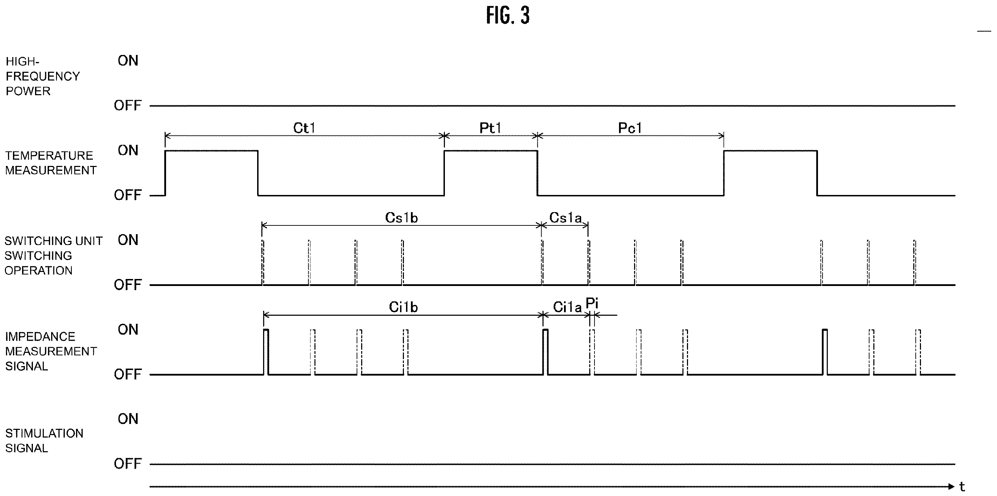

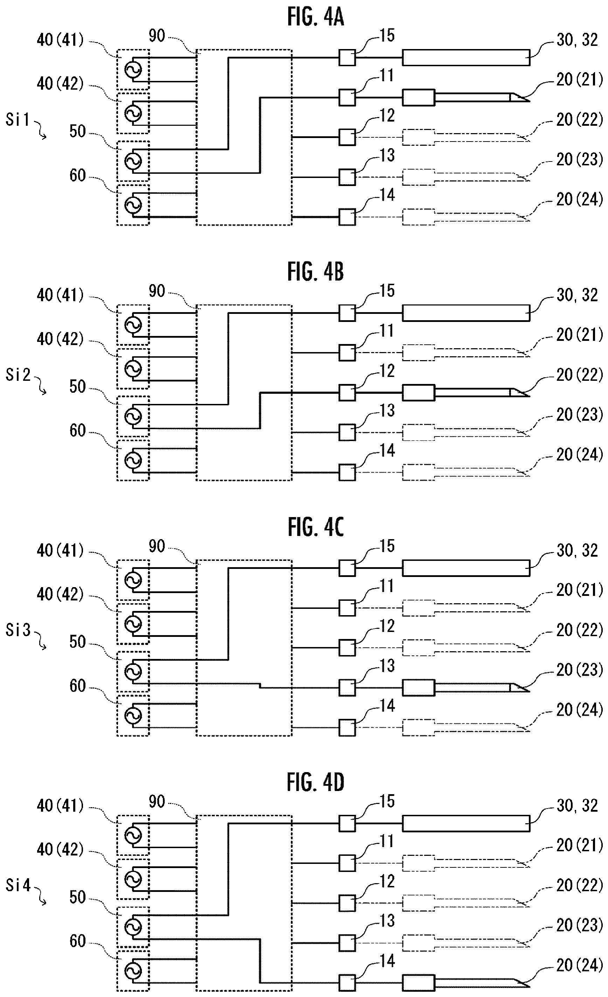

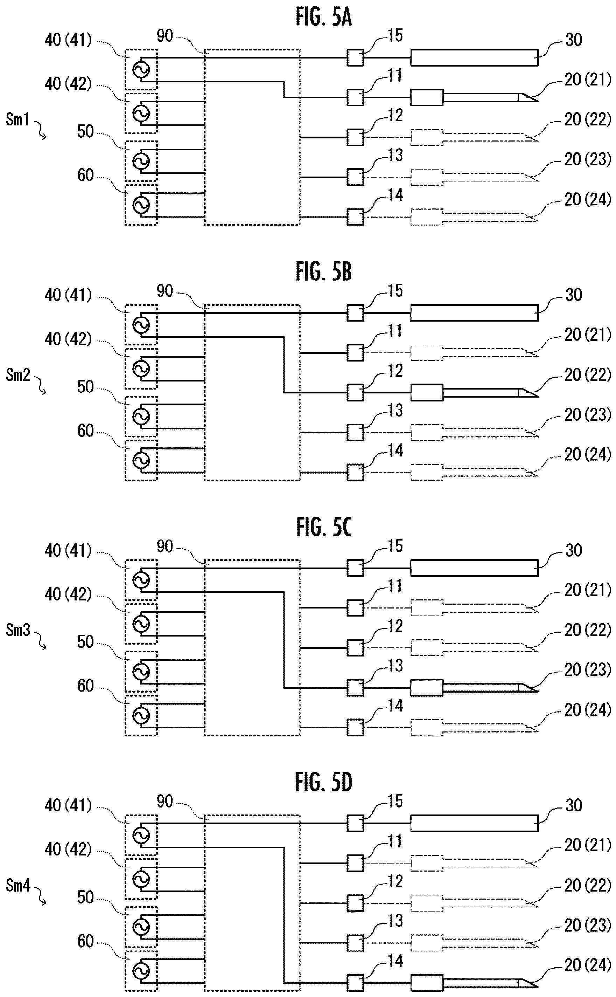

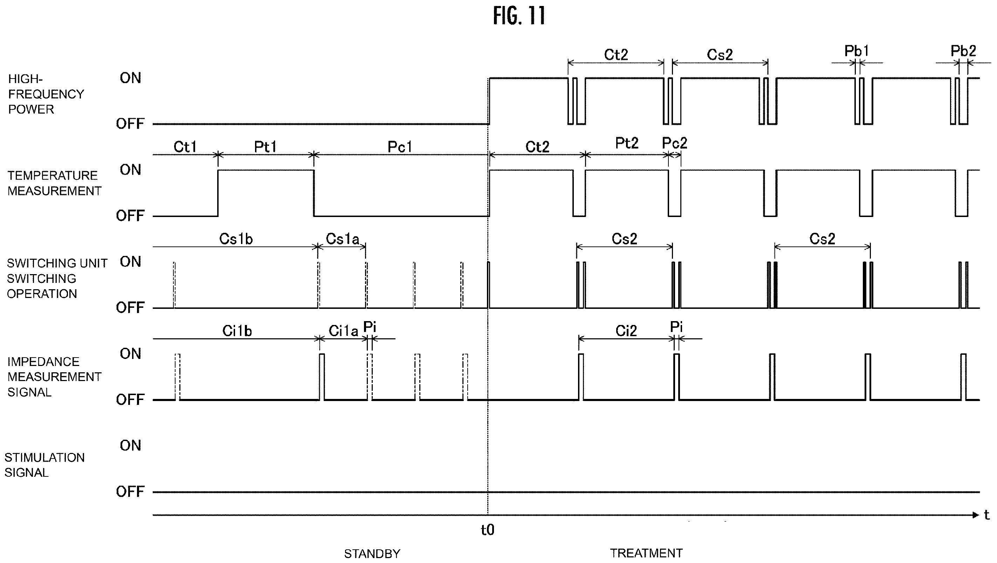

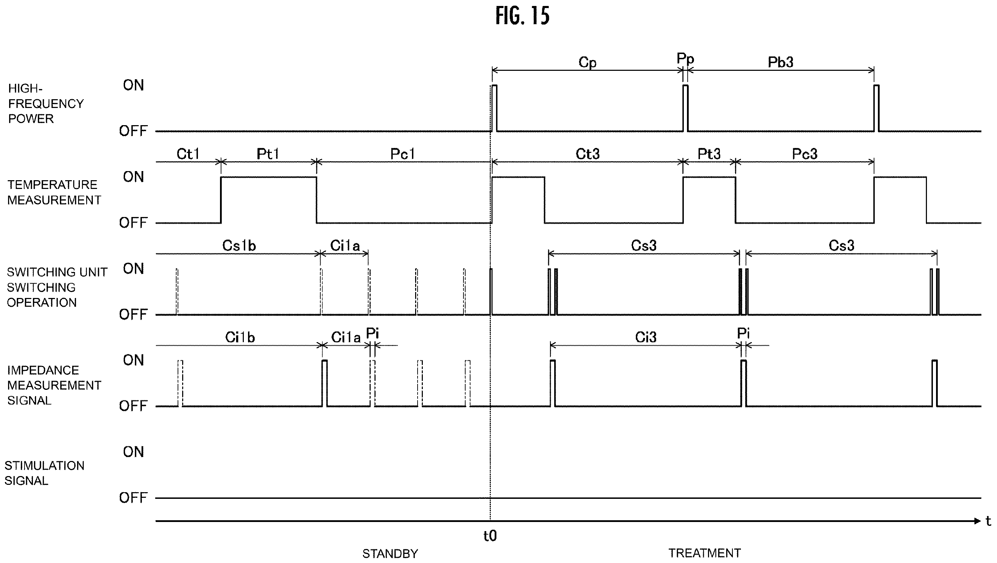

FIG. 3 is a time chart showing an outline of the operation of the high-frequency treatment apparatus 1 in the menu mode, and a time t is indicated on the horizontal axis. FIGS. 4 A-D are schematic diagrams showing the connection state in the menu mode. In FIG. 3, an operation that is canceled depending on the number of connected electrodes 20 is shown by a two-dot chain line (also in FIGS. 11 and 15). Further, in FIGS. 4 A to 4 D, an electrode 20 that does not exist depending on the number of connected electrodes 20 is shown by a two-dot chain line (Same in Figures 5 A-D, 6 A-D and 12 A and B).

In the menu mode, the sub-control unit 102 performs temperature measurement by the temperature measurement unit 70 for each of the connected electrodes 20 at a period Ct1(In this embodiment, 300msec). This temperature measurement is performed at once for all of the connected electrodes 20 by connecting the temperature measurement unit 70 to the thermocouple 20c during a temperature measurement period Pt1(In this embodiment, 100msec). The user can confirm whether the temperature measurement is properly performed based on a change in the temperature measurement value when his/her finger is brought into contact with the electrode 20.

The sub-control unit 102 also measures the impedance between the electrode 20 and the counter electrode plate 30 by the impedance measuring unit 50 for each connected electrode 20 in the menu mode. This impedance measurement is performed by causing the impedance measurement unit 50 to intermittently output an impedance measurement signal with a period Ci1a(In this embodiment, 50msec) and an output period Pi(In this embodiment, 5msec), and causing the switching unit 90 to switch the electrodes 20 connected to the impedance measurement unit 50 with a period Cs1a identical to the period Cila. The impedance measurement is performed during a non-connection period Pc1(In this embodiment, 200msec) when the temperature measurement unit 70 is not connected to the thermocouple 20c in order to eliminate an influence on the temperature measurement.

The switching of the connection states in the menu mode is performed in such a manner that the electrodes 20 through which the impedance measurement signal flows with the counter electrode plate 30 are sequentially changed. That is, for example, when 4 electrodes 20 are connected, the connection state Si1 (FIG. 4 A) in which the impedance measurement signal flows between the electrodes 21 and the counter electrode plate 30, the connection state Si2 (FIG. 4B) in which the impedance measurement signal flows between the electrodes 22 and the counter electrode plate 30, the connection state Si3 (FIG. 4 C) in which the impedance measurement signal flows between the electrodes 23 and the counter electrode plate 30, and the connection state Si4 (FIG. 4D) in which the impedance measurement signal flows between the electrodes 24 and the counter electrode plate 30 are sequentially switched during the non-connection period Pc1.

Further, the sub-control unit 102 changes the number of times the impedance measurement signal is output and changes the mode of switching the connection state in accordance with the number of connected electrodes 20. Specifically, for example, when only the electrodes 21 are connected, the connection state Si1 is maintained in the menu mode (no switching) and the impedance measurement signal is output only once during the non-connection period Pc1. Further, for example, when only the electrodes 21 and 22 are connected, the impedance measurement signal is output 2 times during the non-connection period Pc1 and the connection states Si1 and Si2 are sequentially switched in accordance with this. Further, for example, when only the electrodes 21-23 are connected, the impedance measurement signal is output 3 times during the non-connection period Pc1 and the connection states Si1, Si2, and Si3 are sequentially switched in accordance with this.

As shown in FIG. 3, the output of the impedance measurement signal is started at the same timing during the non-connection period Pc1 regardless of the number of connected electrodes 20, and if the output is performed as many times as the number of connected electrodes 20, the subsequent output is canceled. Further, the switching operation of the switching unit 90 is also canceled in accordance with the number of connected electrodes 20.

As described above, in this embodiment, impedance measurement is periodically performed for all the connected electrodes 20 during the menu mode. Since measurement is performed for all the connected electrodes 20 during the non-connection period Pc1, the measurement period for each electrode 20 is the same period Ci1b as the period Ct1 regardless of the number of connected electrodes 20. Further, the switching period of the connection state for each electrode 20 is the same period Cs1b as the period Ct1.

In this embodiment, output of the impedance measurement signal and switching of the connection state in the menu mode are always performed after detection of connection of the electrodes 20, regardless of whether or not the counter electrode 30 is connected. When an impedance measurement signal flows between the electrode 20 and the counter electrode connector 15, the impedance measurement unit 50 calculates the values of the real and imaginary parts of the complex impedance based on the impedance measurement signal, and transmits the calculated values to the sub-control unit 102. That is, impedance measurement is performed.

Therefore, in the menu mode, the user can check the continuity of the electrode 20 by connecting the test plate 32 to the counter electrode connector 15 instead of the counter electrode 30 and short-circuiting the non-insulating portion 20a of the electrode 20 by contacting the test plate 32. Specifically, when the impedance measurement value becomes a value indicating a short-circuited state, the main control unit 101 outputs an alarm buzzer sound to the speaker, and the user can confirm that the electrode 20 is not disconnected by the output of the alarm buzzer sound.

In the menu mode, after connecting the electrode 20 and the counter electrode 30, the output format and the output mode are set based on an input operation to the touch panel display 16a. The user can select one of the so-called monopolar, bipolar, tripolar, and quad-polar output formats. The user can also select one of the stimulation mode for outputting a stimulation signal to perform nerve exploration, the region mode for outputting a high-frequency power to perform a high-frequency thermocoagulation method, and the PRF mode for outputting a high-frequency power to perform a pulse high-frequency method.

In this embodiment, when any of the bipolar, tripolar, and quad-polar output formats is selected as the output format, only the region mode is selected as the output mode. That is, the bipolar, tripolar, and quad-polar output formats can be used only in the region mode, and only the monopolar output format can be used in the stimulation mode and the PRF mode.

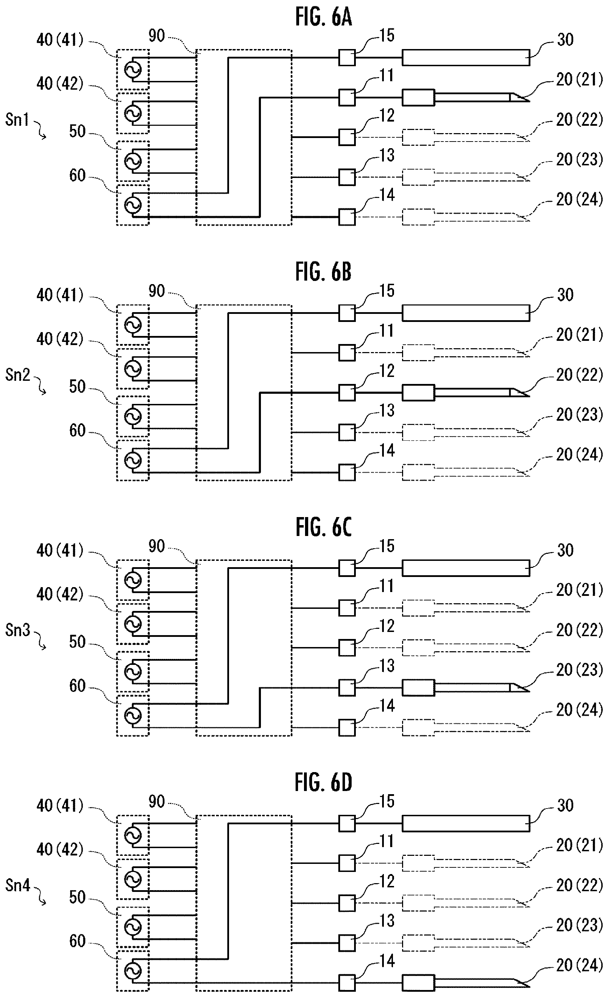

FIGS. 5A-D and 6A-D are schematic diagrams showing connection states in the monopolar. The monopolar is an output type in which a high-frequency current or a stimulation signal flows between one electrode 20 and the counter electrode plate 30. In this embodiment, since 4 electrode connectors 11-14 are provided, the connection state of the monopolar in the region mode and the PRF mode is one of the connection states Sm1 to Sm4 shown in FIGS. 5A-D. Further, since the nerve exploration is performed for each electrode 20, the connection state of the monopolar in the stimulation mode is one of the connection states Sn1 to Sn4 shown in FIGS. 6A-D.

Note that the treatment by the monopolar can be performed successively for each of a plurality of electrodes 20 connected in advance. Therefore, as shown by a two-dot chain line in FIGS. 5A-D, in the connection states Sm1 to Sm4, the electrodes 20 through which the high-frequency current does not flow (the treatment is not performed) may be connected. Similarly, in the connection states Sn1 to Sn4, as shown by a two-dot chain line in FIGS. 6A-D, the electrodes 20 through which the stimulation signal does not flow (the nerve exploration is not performed) may be connected.

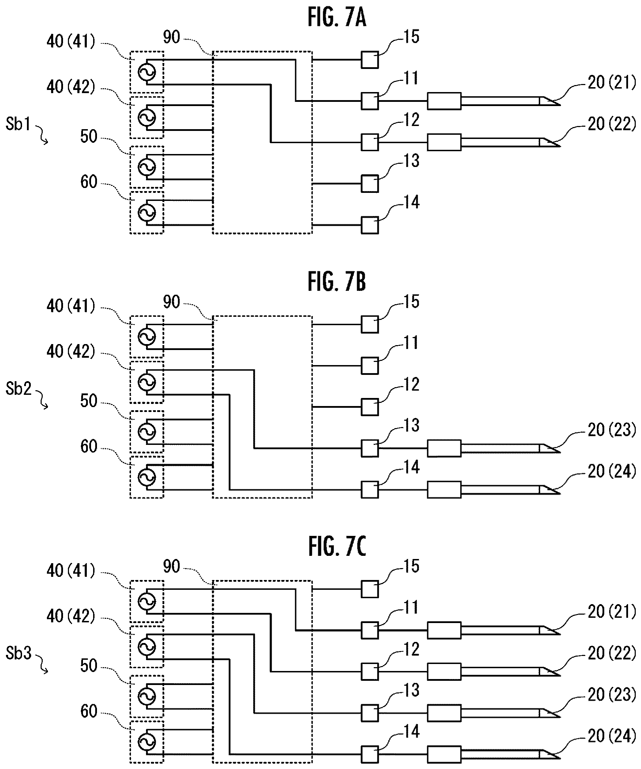

FIGS. 7A-C are schematic diagrams showing the connection states of the bipolar. The bipolar is an output type in which a high-frequency current flows between one electrode 20 and one electrode 20. In the present embodiment, the bipolar can be selected from one of a type in which a current flows between the electrodes 21 and 22 using the first high-frequency output section 41 (connection state Sb1 shown in FIG. 7A), a type in which a current flows between the electrodes 23 and 24 using the second high-frequency output section 42 (connection state Sb2 shown in FIG. 7 B), and a type in which a high-frequency current flows simultaneously between the electrodes 21 and 22 and between the electrodes 23 and 24 using both the first high-frequency output section 41 and the second high-frequency output section 42 (connection state Sb3 shown in FIG. 7C).

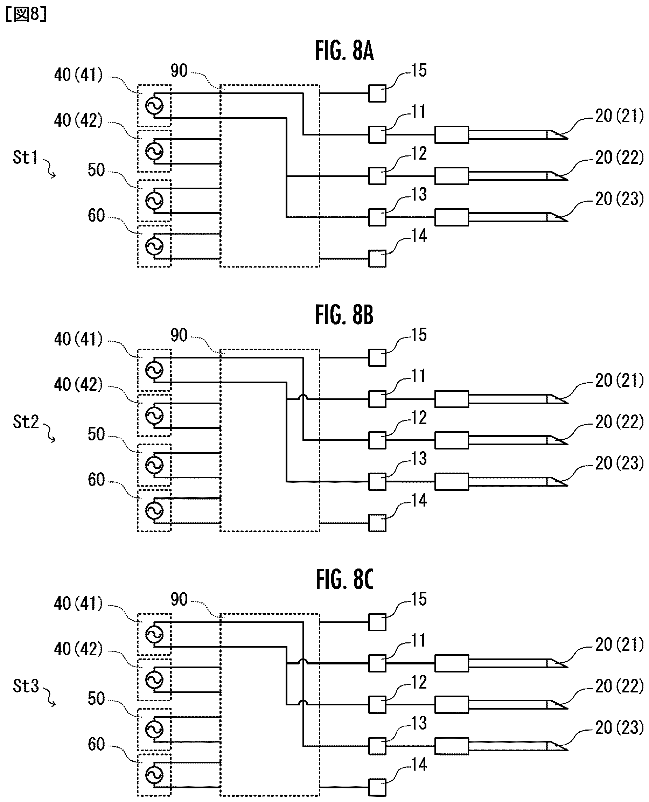

FIGS. 8A-C are schematic diagrams showing the connection states of the tripolar. The tripolar is an output format in which a high-frequency current flows between one reference electrode 20 and the remaining 2 electrodes 20 using the first high-frequency output section 41 and 3 electrodes 20 (electrodes 21-23). In the tripolar, the reference electrode 20 is sequentially changed at a predetermined period Cs2(In this embodiment, 100msec). Specifically, the connection state St1 shown in FIG. 8 A, the connection state St2 shown in FIG. 8B, and the connection state St3 shown in FIG. 8 C are sequentially switched at a period Cs2.

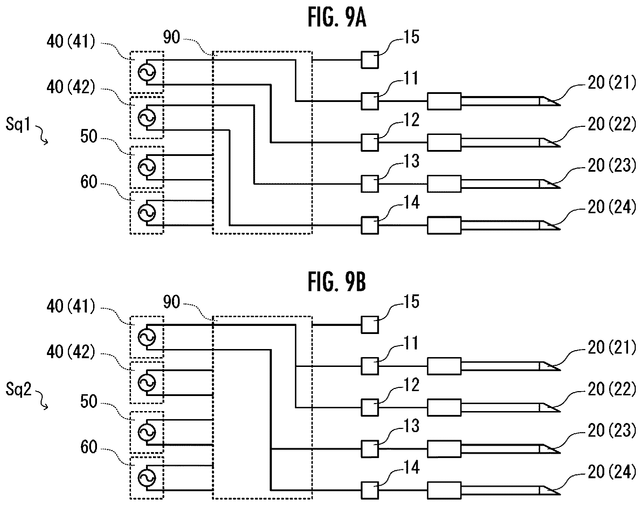

FIGS. 9 A and 9 B are schematic diagrams showing the connection states in the quadpolar. The quad-polar output is an output type that switches between a state in which a high-frequency current flows simultaneously between the electrode 21 and the electrode 22 and between the electrode 23 and the electrode 24 using both the first high-frequency output section 41 and the second high-frequency output section 42 and a state in which a high-frequency current flows between the electrode 21 and the electrode 22 and between the electrode 23 and the electrode 24 using only the first high-frequency output section 41 at a predetermined period Cs2. Specifically, the connection state Sq1 shown in FIG. 9A and the connection state Sq2 shown in FIG. 9B are sequentially switched at a period Cs2.

In the selection of the output mode in the menu mode, the stimulation mode is usually selected first. That is, the nerve exploration is usually performed before the treatment by the high-frequency thermocoagulation method or the pulsed high-frequency method. When the monopolar is selected as the output format and the stimulation mode is selected as the output mode by the operation of the user, the main control unit 101 shifts to the stimulation mode.

In the stimulation mode, the electrode 20 to be used for the nerve exploration, the frequency of the stimulation signal, the pulse width, the output voltage, and the output current are set based on the input operation to the touch panel display 16a while waiting before starting the nerve exploration (before starting the output of the stimulation signal). Further, the user places the electrode 20 and the counter electrode plate 30 on the treatment target during standby. Then, based on the start operation received by the start button 16b or the control knob 16d, the main control unit 101 causes the stimulation signal output unit 60 to start outputting the stimulation signal to start nerve exploration.

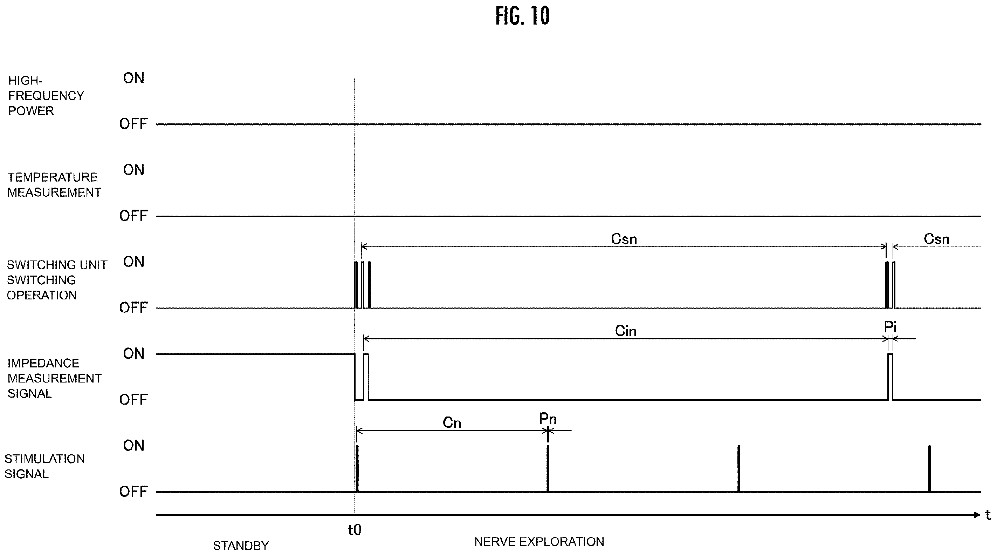

FIG. 10 is a time chart outlining the operation of the high-frequency treatment apparatus 1 in the stimulation mode, and a time t is indicated on the horizontal axis. During standby in the stimulation mode, the sub-control unit 102 stops the temperature measurement by the temperature measurement unit 70. The sub-control unit 102 also causes the switching unit 90 to maintain one of the connection states Si1 to Si4 (For example, connected state Si3 when electrode 23 is used) (FIGS. 4 A to 4 D) depending on which of the electrodes 21-24 is used for nerve exploration, and causes the impedance measurement unit 50 to continuously output the impedance measurement signal. The sub-control unit 102 then calculates the impedance measurement value at a predetermined period (6 msec in this embodiment) and transmits it to the main control unit 101.

The main control unit 101 causes the touch panel display 16a to display the received impedance measurement value, and causes a speaker to output a buzzer sound that changes in accordance with the impedance measurement value. Thus, when the user inserts the electrode 20 into the treatment object and places the electrode 20at the target position, the user can recognize the change in the assumed impedance value not only with the eyes but also with the ears, so that the position of the non-insulated portion 20a at the tip can be more easily estimated.

When the main control unit 101 receives the start operation at an arbitrary time t0, the sub-control unit 102 causes the switching unit 90 to switch the connection to the electrode 20 and the counter electrode 30 from the impedance measurement unit 50 to the stimulation signal output unit 60. That is, for example, when the electrode 23 is used, the connection state Si3 is switched to the connection state Sn3 (FIG. 6C).

The sub-control unit 102 causes the stimulation signal output unit 60 to start outputting the stimulation signal at a timing when the switching from the connection states Si1 to Si4 to the connection states Sn1 to Sn4 is completed. Thus, nerve exploration is started. The stimulation signal is intermittently output with a period Cn (reciprocal of the set frequency) and an output period Pn (2 times the set pulse width).

The sub-control unit 102 also causes the continuous output of the impedance measurement signal from the impedance measurement unit 50 to be changed to intermittent output with a period Cin(In this embodiment, 550msec) and an output period Pi (5msec) when the main control unit 101 receives the start operation. The sub-control unit 102 causes the switching unit 90 to switch the connection to the electrode 20 and the counter electrode 30 from the stimulation signal output unit 60 to the impedance measurement unit 50 at a timing before the output period Pi (connection state Sn1 to Sn4 → connection state Si1 to Si4) and to switch the connection to the electrode 20 and the counter electrode 30 from the impedance measurement unit 50 to the stimulation signal output unit 60 at a timing after the output period Pi (connection state Si1 to Si4→ connection state Sn1 to Sn4). As a result, impedance measurement is performed with a period Cin even during nerve exploration.

During nerve exploration, the main control unit 101 causes the touch panel display 16a to display the impedance measurement value received from the sub-control unit 102. Therefore, the user can discover defects such as the falling of the electrode 20 or the peeling of the counter electrode 30 during nerve exploration based on the impedance measurement value displayed on the touch panel display 16a.

It should be noted that, in the present embodiment, the period Cin is set to 550msec so that the timing of the switching operation of the switching unit 90 and the output of the impedance measurement signal does not overlap with the output timing of the stimulation signal even when either the frequency (period Cn) or the pulse width (output period Pn) of the stimulation signal that can be set is set. However, if the timing does overlap, the output of the stimulation signal is canceled by the sub-control unit 102.

As a result of nerve exploration, it is confirmed that the electrode 20 is placed at an appropriate position. That is, it is confirmed that the electrode 20 is disposed at a position where the sensory nerve can be treated without affecting the motor nerve in the high-frequency treatment to be performed subsequently.

After completion of the nerve exploration, the main control unit 101 returns to the menu mode based on an input operation to the touch panel display 16a. In the menu mode, if the region mode is selected together with any output format by a user's operation, the main control unit 101 shifts to the region mode. In the region mode, the set time and the set temperature are set based on an input operation to the touch panel display 16a while waiting before starting the treatment (before starting the output of the high-frequency power). Then, based on the reception of the start operation by the start button 16b or the control knob 16d, the main control unit 101 causes the high-frequency output unit 40 to start the output of the high-frequency power and starts the treatment.

FIG. 11 is a time chart showing an outline of the operation of the high-frequency treatment apparatus 1 in the region mode, and a time t is indicated on the horizontal axis. In FIG. 11, an operation that is canceled depending on the number of connected electrodes 20 is indicated by a two-dot chain line.

While the region mode is on standby, temperature measurement and impedance measurement are continuously performed in the same manner from the menu mode. Specifically, the sub-control unit 102 performs temperature measurement by the temperature measurement unit 70 for all of the connected electrodes 20 during a period Ct1 (300msec) and a temperature measurement period Pt1 (100msec). The sub-control unit 102 also causes the impedance measurement unit 50 to output an impedance measurement signal during a period Ci1a (50msec) and an output period Pi (5msec) in accordance with the number of electrodes 20 for each non-connection period Pc1, and causes the switching unit 90 to switch the connection states Si1 to Si4 during a period Cs1a that is the same as the period Ci1a, thereby measuring the impedance between the electrodes 20 and the counter electrode plate 30 for each electrode 20.

Therefore, when the output type is monopolar using the counter electrode 30, the impedance measurement during standby continues from the menu mode. On the other hand, when the output type is bipolar, tripolar or quad-polar not using the counter electrode 30, the impedance measurement during standby is not performed after the counter electrode 30 is removed during standby.

It should be noted that when the output type is bipolar, tripolar or quad-polar, the main control unit 101 causes the touch panel display 16a to display a guide display prompting removal of the counter electrode 30 during standby. The output of the impedance measurement signal by the impedance measurement unit 50 and the switching operation of the switching unit 90 are performed even after the counter electrode 30 is removed, which is the same as in the menu mode.

The main control unit 101 causes the touch panel display 16a to display the temperature measurement value and the impedance measurement value measured during standby. Therefore, when the output type is monopolar, the user can discover a failure such as the falling of the electrode 20 or the peeling of the counter electrode 30 based on the impedance measurement value displayed on the touch panel display 16a.

In addition, in this embodiment, the frequency of the sine wave of the impedance measurement signal is set to 50 kHz and the output period Ci1 is set to 300msec, so that the impedance measurement signal also functions as a stimulation signal for motor nerve exploration. Thus, in this embodiment, when the output type is monopolar, the user can discover that the electrode 20 is moved to a position that affects the motor nerve due to an unintended displacement or the like after the nerve exploration. It is also possible to omit the exploration of the motor nerve in the nerve exploration in advance.

It should be noted that even when the output type is bipolar, tripolar, or quad-polar, the above-described effects can be obtained during standby while the counter electrode plate 30 is disposed as a treatment object following the nerve exploration.

As shown in FIG. 11, when the main control unit 101 receives the start operation at an arbitrary time t0, the sub-control unit 102 causes the switching unit 90 to switch the connection to the electrode 20 and the counter electrode 30 from the impedance measuring unit 50 to the high-frequency output unit 40.

Specifically, when the output type is monopolar, the sub-control unit 102 causes the switching unit 90 to switch from any of the connection states Si1 to Si4 (FIGS. 4A to 4D) to any of the connection states Sm1 to Sm4 (FIGS. 5A to 5D). When the output type is bipolar, the sub-control unit 102 causes the switching unit 90 to switch from any of the connection states Si1 to Si4 to any of the connection states Sb1 to Sb3 (FIGS. 7A to 7C) corresponding to the number and connection position of the electrodes 20. When the output type is tripolar, the sub-control unit 102 causes the switching unit 90 to switch from any of the connection states Si1 to Si4 to the connection state St1 (FIG. 8A). When the output type is quad-polar, the sub-control unit 102 causes the switching unit 90 to switch from any of the connection states Si1 to Si4 to the connection state Sq1 (FIG. 9A).

The main control unit 101 causes the first high-frequency output unit 41 or the second high-frequency output unit 42 connected to the electrodes 20 to start outputting high-frequency power at a timing at which the switching of the connection states is completed. Thus, the treatment is started.

For outputting high-frequency power in the region mode, a first stop period Pb1 (5msec) is provided at a cycle Ct2(In this embodiment, 100msec) of temperature measurement by the temperature measurement unit 70 so as to avoid the influence of the high-frequency power on temperature measurement. Then, the sub-control unit 102 starts connecting the temperature measurement unit 70 and the thermocouple 20c at the same timing as the start of outputting high-frequency power, and measures temperatures of all the connected electrodes 20 during the first stop period Pb1. In this embodiment, the temperature measurement period Pt2 during the treatment in the region mode is set to 87msec. Therefore, the period from the start of the output of the high-frequency power to the first stop period Pb1 is 82msec. The non-connection period Pc2 in which the temperature measuring section 70 and the thermocouple 20c are not connected during the treatment is 13msec.

For the output of the high-frequency power, a second stop period Pb2(In this embodiment, 9msec) is provided at a timing 4msec after the first stop period Pb1 in the non-connection period Pc2 with a period Cs2 (100msec) for switching the connection state in the tri-polar and quad-polar systems. Since the period Cs2 is the same as the period Ct2, the interval between the first stop period Pb1 and the second stop period Pb2 is maintained at 4msec.

The sub-control section 102 causes the switching section 90 to switch the connection state and the impedance measuring section 50 to measure the impedance during the second stop period Pb2. Therefore, during the treatment, the sub-control section 102 causes the impedance measuring section 50 to intermittently output an impedance measurement signal with a period Ci2 (100msec) and an output period Pi (5msec) identical to the period Cs2 (and the period Ct2).

The sub-control unit 102 also causes the switching unit 90 to switch the connections to the electrode 20 and the counter electrode plate 30 from the high-frequency output unit 40 to the impedance measurement unit 50 at a timing before the output period Pi and to switch the connections to the electrode 20 and the counter electrode plate 30 from the impedance measurement unit 50 to the high-frequency output unit 40 at a timing after the output period Pi at a period Cs2 (100msec). That is, during the treatment in the region mode, the impedance is measured for a combination of the electrode 20 and the counter electrode plate 30 through which a high-frequency current flows or a combination of the electrode 20 through which a high-frequency current flows.

When the output type is monopolar, the connection state when the impedance is measured during the treatment is one of the connection states Si1 to Si4 (FIGS. 4A to 4D) identical to those in the menu mode (and standby). When the output type is monopolar and the electrode 21 is used, for example, the sub-control unit 102 causes the switching unit 90 to switch from the connection state Sm1 (FIG. 5A) to the connection state Si1 (FIG. 4A) before the output period Pi and causes the switching unit 90 to switch from the connection state Si1 to the connection state Sm1 after the output period Pi. That is, when the output type is monopolar, the impedance during the treatment is measured only for the electrode 20 used (through which the high-frequency current flows) even if a plurality of electrodes 20 are connected.

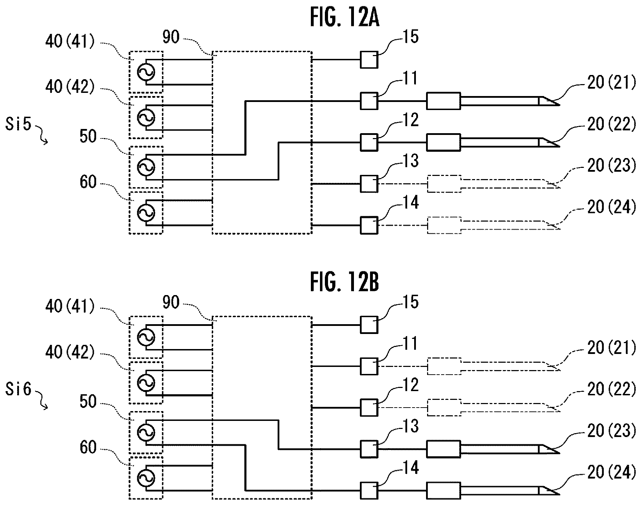

FIGS. 12A and B are schematic diagrams illustrating the state of connection during impedance measurement during bipolar treatment. When the output type is bipolar and only the electrodes 21 and 22 are connected, the sub-control unit 102 causes the switching unit 90 to switch from the connection state Sb1 (FIG. 7A) to the connection state Si5 before the output period Pi, and causes the switching unit 90 to switch from the connection state Si5 to the connection state Sb1 after the output period Pi. When the output type is bipolar and only the electrodes 23 and 24 are connected, the sub-control unit 102 causes the switching unit 90 to switch from the connection state Sb2 (FIG. 7B) to the connection state Si6 before the output period Pi, and causes the switching unit 90 to switch from the connection state Si6 to the connection state Sb2 after the output period Pi.

When the output type is bipolar and all of the electrodes 21-24 are connected, the sub-control unit 102 causes the switching unit 90 to switch from the connection state Sb3 (FIG. 7C) to the connection state Si5 or Si6 before the output period Pi, and causes the switching unit 90 to switch from the connection state Si5 or Si6 to the connection state Sb3 after the output period Pi. It should be noted that switching from the connection state Sb3 to either the connection state Si5 or the connection state Si6 is alternately changed each time the switching is performed. Therefore, impedance measurement in this case is performed by alternately changing the connection states Si5 and Si6 in the period Cs2.

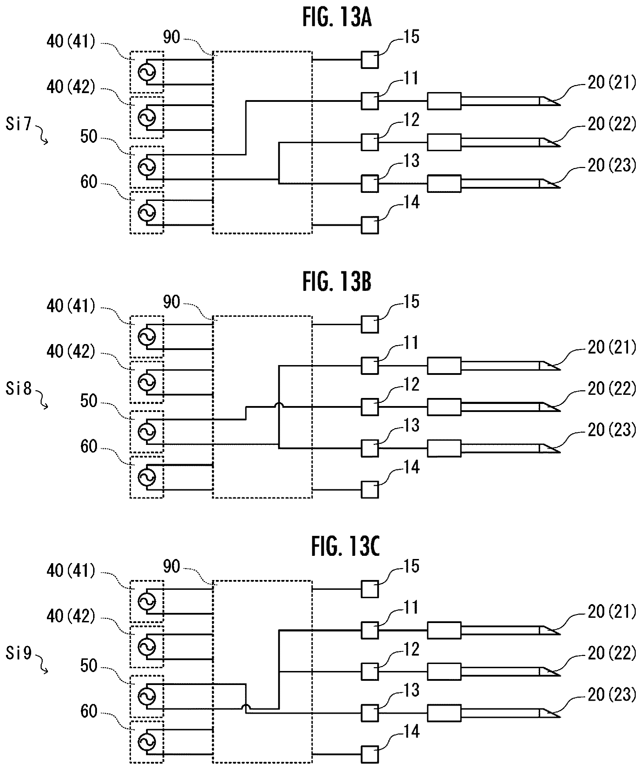

FIGS. 13A to C are schematic diagrams showing connection states during impedance measurement during treatment with the tripolar device. When the output format is the tripolar device, the sub-control unit 102 causes the switching unit 90 to switch from the connection state St1 (FIG. 8A) to the connection state Si7, from the connection state St2 (FIG. 8B) to the connection state Si8, or from the connection state St3 (FIG. 8C) to the connection state Si9 before the output period Pi in accordance with the connection state just before. The sub-control unit 102 also causes the switching unit 90 to switch from the connection state Si7 to the connection state St2, from the connection state Si8 to the connection state St3, or from the connection state Si9 to the connection state St1 after the output period Pi in accordance with the connection state just before.

Therefore, when the output type is tripolar, impedance measurement during the treatment is performed by sequentially changing the connection states Si7 to Si9 in the period Cs2. Switching between the connection states St1 to St3 in the period Cs2 is performed by switching after the output period Pi.

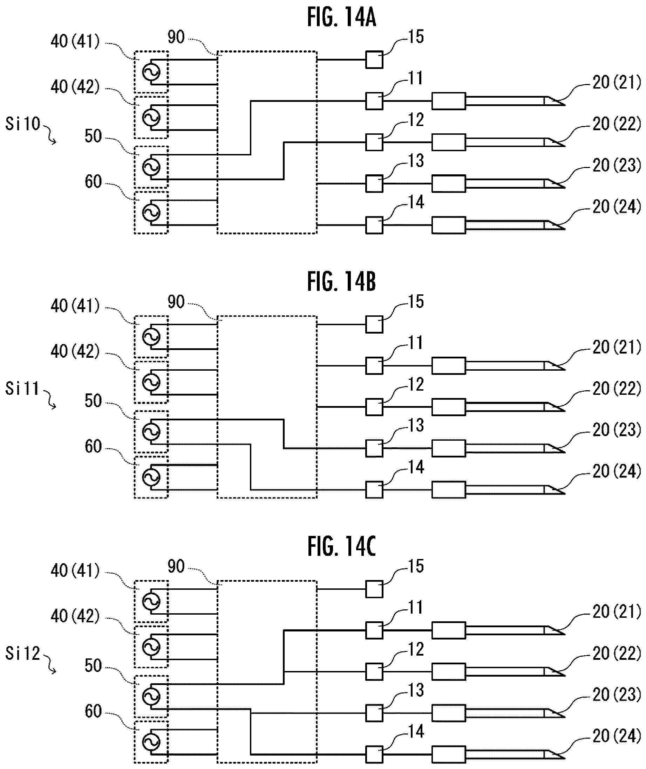

FIGS. 14A-C are schematic diagrams illustrating the state of connection during impedance measurement during quad-polar treatment. When the output type is quad-polar, the sub-control unit 102 causes the switching unit 90 to switch from the connection state Sq1 (FIG. 9A) to the connection state Si10 or Si11 or from the connection state Sq2 (FIG. 8C) to the connection state Si12 before the output period Pi in accordance with the connection state immediately before. It should be noted that switching between the connection states Si11 and Si12 from the connection state Sq1 is alternately changed each time the switching is performed. The sub-control unit 102 also causes the switching from the connection state Si10 or Si11 to the connection state Sq2 or from the connection state Si12 to the connection state Sq1 after the output period Pi in accordance with the connection state immediately before.

Therefore, when the output type is quad-polar, the impedance measurement during the treatment is performed by changing the connection state in the order of connection state Si10→ connection state Si12→ connection state Si11→ connection state Si12 in the period Cs2. The switching between the connection states Sq1 and Sq2 in the period Cs2 is performed by switching after the output period Pi.

The switching between the connection states St1 to St3 in the period Cs2 when the output type is tri-polar and the switching between the connection states Sq1 and Sq2 in the period Cs2 when the output type is quad-polar can also be performed by switching before the output period Pi.

In this way, even during the treatment, the sub-control unit 102 causes the impedance measurement unit 50 to periodically measure the impedance for each combination of the electrode 20 and the counter electrode 30 through which the high-frequency current flows when the output type is mono-polar and for each combination of the electrode 20 through which the high-frequency current flows when the output type is bipolar, tri-polar or quad-polar.

The main control unit 101 causes the touch panel display 16a to display the temperature measurement value and the impedance measurement value as well as the output voltage and the output current of the high-frequency power during the treatment. Therefore, based on the impedance measurement value displayed on the touch panel display 16a, the user can discover defects such as the disconnection of the electrode 20 or the peeling of the counter electrode plate 30.

Further, the main control unit 101 causes the touch panel display 16a to display the elapsed time as a countdown display during the treatment, and stops the output of the high-frequency power to the high-frequency output unit 40 when the set time has elapsed.

In this embodiment, the intermittent output of the impedance measurement signal from the impedance measurement unit 50 is continuously performed during the treatment from the standby state, so that the impedance measurement unit 50 can measure the impedance with high accuracy even during the treatment. That is, in conventional apparatuses, the impedance during the treatment is measured by calculating the impedance from the voltage measurement value and the current measurement value of the high-frequency power, so that there is a problem that an inaccurate impedance is easily calculated due to the difficulty of the high-frequency current direct measurement with high accuracy. However, according to this embodiment, the impedance can be measured with high accuracy even during the treatment.

The high-accuracy measurement of the impedance makes it possible for the user to detect problems occurring during the treatment at an early stage from the impedance measurement value, so that the treatment can be performed safely. Furthermore, since the output current of the high-frequency power can be measured with high accuracy by calculating it from the impedance measurement value, the user can accurately grasp the execution state of the treatment.

Furthermore, according to this embodiment, the period Ci2 of the output of the impedance measurement signal during the treatment is changed from the period Ci1 during the standby state, and is made to coincide (synchronize) with the period Cs2 of the connection state switching in the tri-polar and quad-polar apparatuses and the period Ct2 of the temperature measurement, so that the impedance measurement unit 50 can efficiently measure the impedance without interfering with them.

When the PRF mode is selected by the user's operation together with any of the output formats in the menu mode after the completion of the nerve exploration, the main control unit 101 shifts to the PRF mode. In the PRF mode, during the standby before starting the treatment (before starting the output of the high-frequency power), the termination method (completion of the set time or the set number of pulses), the set time or the set number of pulses, the set temperature, the set pulse frequency, the set pulse width, and the set output voltage are set based on the input operation to the touch panel display 16a. Then, based on the reception of the start operation by the start button 16b or the control knob 16d, the main control unit 101 causes the high-frequency output unit 40 to start outputting the high-frequency power to start the treatment.

FIG. 15 is a time chart showing an outline of the operation of the high-frequency treatment apparatus 1 in the PRF mode, and a time t is indicated on the horizontal axis. During standby in the PRF mode, temperature measurement and impedance measurement are continuously performed from the menu mode. Since the output format in the PRF mode is only monopolar, temperature measurement and impedance measurement during standby are performed in the same manner as in the menu mode.

The main control unit 101 causes the touch panel display 16a to display the temperature measurement value and impedance measurement value measured during standby. Therefore, based on the impedance measurement value, the user can discover defects such as the falling of the electrode 20 or the peeling of the counter electrode plate 30.

Further, as described above, in the present embodiment, since the impedance measurement signal also functions as a stimulation signal for motor nerve exploration, the user can discover defects such as the unexpected displacement of the electrode 20 after nerve exploration even during standby in the PRF mode. It is also possible to omit the exploration of the motor nerve in the nerve exploration in advance.

As shown in FIG. 15, when the main control unit 101 receives the start operation at an arbitrary time t0, the sub-control unit 102 causes the switching unit 90 to switch the connection to the electrode 20 and the counter electrode plate 30 from the impedance measurement unit 50 to the first high-frequency output unit 41. Specifically, the sub-control unit 102 causes the switching unit 90 to switch from one of the connection states Si1 to Si4 (FIGS. 4A-D) to one of the connection states Sm1 to Sm4 (FIGS. 5A-D).

Then, the main control unit 101 causes the first high-frequency output unit 41 to start outputting the high-frequency power at the timing when the switching of the connection state is completed. Thus, the treatment is started.

As shown in FIG. 15, the output of the high-frequency power in the PRF mode is intermittently performed at a period Cp (inverse of the set pulse frequency) and an output period Pp (set pulse width). In this embodiment, the period Cp is selected from 1000msec (1 Hz), 500msec (2 Hz), 200msec (5 Hz), and 100msec (10 Hz). The output period Pp is selected from 5msec, 10msec, 20msec, 30msec, and 50msec.

The sub-control unit 102 starts the connection between the temperature measuring unit 70 and the thermocouple 20c at the same timing as the start of the output of the high-frequency power, and measures the temperatures of all the connected electrodes 20 during the third stop period Pb3 when the high-frequency power is not output in order to avoid the influence of the high-frequency power on the temperature measurement. Therefore, the temperature measurement during the treatment is performed at the same period Ct3 as the period Cp. In this embodiment, the temperature measurement period Pt3 is set to 55msec, which is longer than the maximum output period Pp. Therefore, the non-connection period Pc3 when the temperature measuring unit 70 and the thermocouple 20c are not connected during the treatment is the difference between the period Cp and the temperature measurement period Pt3.

During the treatment, the sub-control unit 102 causes the impedance measuring unit 50 to intermittently output an impedance measurement signal at the same period Ci3 and output period Pi (5msec) as the period Cp. The sub-control unit 102 also causes the switching unit 90 to switch the connection to the electrode 20 and the counter electrode 30 from the high-frequency output unit 40 to the impedance measurement unit 50 at a timing before the output period Pi, and to switch the connection to the electrode 20 and the counter electrode 30 from the impedance measurement unit 50 to the high-frequency output unit 40 at a timing after the output period Pi, at a period Cs3 which is the same as the period Ci3 (and the period Cp). Specifically, for example, when the electrode 23 is used, switching is performed between the connection state Sm3 (FIG. 5C) and the connection state Si3 (FIG. 4C).

In this way, the sub-control unit 102 causes the impedance measurement unit 50 to periodically measure the impedance of the combination of the electrode 20 and the counter electrode 30 through which the high-frequency current flows, even during the treatment. The switching of the connection state and the output of the impedance measurement signal are performed during the non-connection period Pc3 which is included in the third stop period Pb3.

The main control unit 101 causes the touch panel display 16a to display the temperature measurement value and the impedance measurement value together with the output voltage and the output current of the high-frequency power during the treatment. Therefore, the user can discover defects such as the disconnection of the electrode 20 or the peeling of the counter electrode 30 based on the impedance measurement value displayed on the touch panel display 16a.

The main control unit 101 causes the touch panel display 16a to display the elapsed time or the number of pulses in a countdown display during the treatment, and stops the output of the high-frequency power to the high-frequency output unit 40 when the set time has elapsed or the set number of pulses has been completed.

In the PRF mode, as in the region mode, the intermittent output of the impedance measurement signal from the impedance measurement unit 50 is continuously performed during the treatment from the standby state, so that the impedance measurement is performed with high accuracy even during the treatment. In the PRF mode, the impedance measurement is efficiently performed by making the output cycle Ci3 of the impedance measurement signal during the treatment coincide (synchronize) with the output cycle Cp of the high-frequency power.

Although the embodiments of the present invention have been described above, the high-frequency treatment apparatus and the high-frequency treatment method of the present invention are not limited to the above-described embodiments, and various modifications can be made without departing from the gist of the present invention.

Further, the operations and effects shown in the above-described embodiments are only a list of the most suitable operations and effects resulting from the present invention, and the operations and effects according to the present invention are not limited thereto.
1 High-frequency treatment apparatus

### [Reference Signs List]

20 Electrode
40 High-frequency output unit
50 Impedance measurement unit
100 Control unit
Pb2 second stop period

## Claims

1. A high-frequency treatment apparatus comprising:
a high-frequency output unit outputting high-frequency power;
an electrode connected to the high-frequency output unit and arranged on a treatment object;
an impedance measurement unit connected to the electrode and measuring impedance by outputting an impedance measurement signal;
and a control unit controlling the impedance measurement unit so as to continue outputting the impedance measurement signal intermittently before and after the start of a treatment using the high-frequency power.

2. The high-frequency treatment apparatus according to claim 1, wherein:
before the start of the treatment, the control unit controls the impedance measurement unit so as to output the impedance measurement signal in a manner in which a motor nerve is stimulated.

3. The high-frequency treatment apparatus according to claim 1, wherein:
after the start of the treatment, the control unit controls the impedance measurement unit so as to output the impedance measurement signal in a manner different from a state before the start of the treatment.

4. The high-frequency treatment apparatus according to claim 1, wherein:
the high-frequency output unit intermittently outputs the high-frequency power after starting the treatment;
and the control unit controls the impedance measurement unit so as to output the impedance measurement signal during a stop period of the output of the high-frequency power after starting the treatment.

5. The high-frequency treatment apparatus according to claim 1, further comprising:
an alarm which is controlled by the control unit to issue an alarm;
and the control unit controls the alarm so as to issue an alarm when the impedance measured by the impedance measurement unit is a value indicating a short-circuit state.

6. A high-frequency treatment method performing a treatment by outputting high-frequency power to an electrode arranged in a treatment object, wherein:
intermittent output of an impedance measurement signal for measuring impedance is continued from a state before starting the treatment to a state after starting the treatment.
